# EUROPEAN PATENT APPLICATION

(11) **EP 1 813 279 A1**
(43) Date of publication of application: **01.08.2007**
(21) Application number: 07472001.2
(22) Date of filing: 30.01.2007
(51) Int. Cl.: A61K 33/14, A61K 33/10, A61L 2/02, A61L 2/18, A61L 33/00, A61P 43/00

(54) **Concentrated hemodialysis solutions**

(30) Priority: 30.01.2006 BG 10942206
(71) Applicant: Sopharma AD, 1220 Sofia (BG)
(72) Inventor: Daskalov, Angel Dimotrov, 1113 Sofia (BG); Ionova-Ivancheva, Diana Hristova, Sofia (BG); Chipanov, Dimitar Dimitrov, 1612 Sofia (BG); Antonov, Simeon Antonov, Sofia (BG); Istilyanova, Aneta Yordanova, 1797 Sofia (BG); Georgiev, Mitko Ivanov, Sofia (BG)
(74) Representative: Kostadinova, Rossitsa Kirilova

(57) **Abstract**

The present invention relates to concentrated solutions used to make ready-for-use hemodialysis solutions applicable in patients with acute or chronic renal failure, detoxification, acute intoxication and patients with calcium and lipid metabolism disorders.

The obtained concentrated hemodialysis solutions contain - in addition to ions selected among: sodium, calcium, potassium, magnesium, chorine, acetate, and hydrogen carbonate ions, and glucose - disodium ethylene diamine tetraacetic acid (DS-EDTA) as an active substance.

The ready-for-use hemodialysis solutions are applied for the prevention and treatment of cardiovascular diseases involving atheromatous change and cardiovascular and soft-tissue calcification in chronic renal failure patients on hemodialysis; for the prevention and treatment of calcium body overload in chronic renal failure patients on hemodialysis; for the prevention and treatment of calciphylaxis in chronic renal failure patients on hemodialysis; for the prevention and treatment of renal osteodystrophy (in the presence of hyperphosphataemia and hypercalcaemia) in chronic renal failure patients on hemodialysis; for the prevention and treatment of diabetic macroangiopathy in diabetic patients on hemodialysis; for the treatment of heavy metal intoxication in patients with general renal failure and chronic renal failure.

## Description

### FIELD OF THE INVENTION

The present invention relates to concentrated solutions used to make ready-for-use hemodialysis solutions applicable in patients with acute or chronic renal failure, detoxification after acute intoxication and patients with calcium and lipid metabolism disorders.

### BACKGROUND OF THE INVENTION

Several types of concentrated hemodialysis solutions are currently available for use in conventional hemodialysis. These contain various concentrations of calcium, potassium, sodium, magnesium, chlorine, bicarbonate and/or acetate ions and glucose. [Krushkov, I., Lambev, I., Pharmacotherapeutic Guide, 6th edition, and European Pharmacopoeia 5.0; Haemodialysis, solution for]

For preparation of bicarbonate hemodialysis solutions are used concentrated acid solutions. These contain ions within the following limits:

| **Ions** | **Concentrated solution in mmol/l** |
|---|---|
| Sodium | from 4550.0 to 5075.0 |
| Potassium | from 0.00 to 105.0 |
| Calcium | from 0.00 to 70.0 |
| Magnesium | from 0.00 to 42.0 |
| Chlorine | from 3150.0 to 4200.0 |
| Acetate | from 87.5 to 350.0 |
| Glucose | from 0.00 to 420.0 |

and a basic bicarbonate solution containing a hydrogen carbonate ion from 595.189 to 1050.0 mmol/l . These concentrated solutions are used to prepare ready-for-use hemodialysis solutions in the following ratio: 1 part of concentrated acid solution plus 1.225 parts of basic bicarbonate solution and 32.775 parts of water for hemodialysis, with the following compositions:

| **Ions** | **Ready-for-use solutions in mmol/l** |
|---|---|
| Sodium | from 130.0 to 145.0 |
| Potassium | from 0.00 to 4.0 |
| Calcium | from 0.00 to 2.0 |
| Magnesium | from 0.00 to 1.2 |
| Chlorine | from 90.0 to 120.0 |
| Acetate | from 2.5 to 10.0 |
| Glucose | from 0.00 to 12.0 |
| Hydrogen carbonate | from 19.18 to 36.75 |

Concentrated solutions for acetate hemodialysis contain ions within the following limits:

| **Ions** | **Concentrated solutions in mmol/l** |
|---|---|
| Sodium | from 4550.0 to 5075.0 |
| Potassium | from 00.00 to 105.0 |
| Calcium | from 00.00 to 70.0 |
| Magnesium | from 00.0 to 42.0 |
| Chlorine | from 3150.0 to 4200.0 |
| Acetate | from 1120.0 to 1575.0 |
| Glucose | from 00.00 to 420.0 |

These concentrated solutions are diluted with water for hemodialysis in a 1: 35 ratio to prepare ready-for-use hemodialysis solutions with the following compositions:

| **Ions** | **Ready-for-use solutions in mmol/l** |
|---|---|
| Sodium | from 130.0 to 145.0 |
| Potassium | from 0.00 to 3.0 |
| Calcium | from 0.00 to 2.0 |
| Magnesium | from 0.00 to 1.2 |
| Chlorine | from 90.0 to 120.0 |
| Acetate | from 32.0 to 45.0 |
| Glucose | from 0.00 to 12.0 |

One of the major and unsolved problems in patients with chronic renal failure (CRF) on haemodialysis (HD) is the impaired mineral metabolism. The main factors that maintain and exacerbate this pathology include hyperphosphataemia, increased serum calcium and phosphate levels, intermittent change in alkaline acid balance, secondary hyperparathyroidism or hypoparathyroidism, high-dose administration of calcitriol (the most active metabolite of vitamin D₃ which is physiologically synthesized in the kidney but CRF and HD patients are highly deficient therein), and calcium containing oral phosphate binders inducing calcium body overload. Soft-tissue and, among them, cardiovascular calcium deposits have a special place among symptoms of impaired mineral metabolism. The latter deposits have been definitely identified as a self dependent risk factor increasing the cardiovascular morbidity and mortality (values being manifold higher in CRF and HD patients than those in the general population). According to literature data [Foley RN, Parfey PS, Sarnak MJ; Clinical epidemiology of cardiovascular disease in chronic renal disease. Am J Kidney Dis, 1998;32:5112-19; Fatica RA, Dennis VW; Hyperphosphatemia, coronary calcification and the role of P binders. Cleve Clin J Med 2002 (Suppl.3):S21-S27; Schoonhagen P, Murat Tuzcu E; Coronary artery calcification and ESRD: Vascular biology and clinical implications. Cleve Clin. J Med 2002 (Suppl.3):S12-S20], soft-tissue calcifications were found in 70 to 95 per cent of HD patients which makes solving this problem particularly actual.

Essentially cardiovascular calcifications in CRF and HD patients are two types depending on their nature and localization in the vessel wall:
a). in the atheromatous plaques, they are found in the heart structures and arterial intima bound to the atherogenous lipid fractions,
b). in the so-called mediocalcinosis, they are located extracellularly in the smooth muscle layers of the arteries and heart structures and their composition is similar but not identical to that of the bones. The mineral phase of normal bones is composed of hydroxy apatite, while the pathologic calcific deposits are a mixture of hydroxy apatite, carbonate apatite, and amorphous calcium phosphate, i.e. a defective and non homogenous bone substance with unusual tissue localization (in the cardio-vascular system, skin, muscles, periarticular tissue, eye tissue, and all viscera). In these deposits, calcium ions are located in a defective crystal lattice and they are more loosely bound than those of the natural crystals found in the bone structure.

Calcification itself is a complex and active process involving monocytes and macrophages from the bloodstream making extracellular contacts and excreting active factors and osteogenic proteins after extravasation. The presence of calcium ions is obligatory to trigger the osteogenic potential and intracellular interaction of these cells. Hyperphosphataemia has proven to play an active role in calcium phosphate formation in the cardiovascular structures. Together with some substances secreted by monocytes, macrophages, and vessel wall cells, it transforms soft muscle cells into osteogenic cells.

There is a specific form of pathological calcium phosphate deposits called calciphylaxis, with a particularly dramatic evolution in CRF and HD patients entailing limb amputation. Symptoms include progressive skin necrosis of the limbs due to calcification, inflammatory processes, fibrosis, and obstruction of small and medium blood vessels deep in the skin and subcutis followed by deep tissue necrosis and gangrene. Here again, monocytes and macrophages, which, as mentioned above, require the presence of calcium ions to perform their functions, play an active role in inflammatory change and pathologic osteogenesis with atypical localization.

No effective method has been found so far to reduce or "clear out" cardiovascular and other soft-tissue calcification in CRF and HD patients, although many attempts have been undertaken in this direction through normalization of serum phosphate and calcium levels and treatment of secondary hyperparathyroidism, including parathyroidectomy and renal transplantation. (Partial success was only achieved with great difficulty using the above methods in some soft-tissue localizations other than cardiovascular such as the skin, subcutis, and periarticular spaces).

Also, there is a cohort of patients with impaired lipid metabolism (dyslipoproteinaemia) due to: metabolic syndromes caused by dietary habits and lifestyle, other diseases and some medications (diabetes, nephrotic syndrome, hypothyroidism, cholestasis, renal failure, alcohol addiction, corticosteroid treatment, intake of thiazide diuretics etc.), as well as primary disorders of lipid metabolism.

In all cases of dyslipoproteinaemia described, there is localized infiltration of atherogenous low-density lipoproteins into the cardiovascular walls and atheromatous plaques formation with subsequent calcification, i.e. development of atherosclerosis - a condition that has well known severe clinical implications for the patient.

CRF and HD patients are at high risk of dyslipoproteinaemia and atherosclerosis for nearly all underlying causes described above. CRF and HD patients with diabetes mellitus and diabetic nephropathy develop early and severe atherosclerosis (the so-called diabetic macroangiopathy) due to marked metabolic syndromes of carbohydrate and lipid metabolism related to the main disease.

Concentrates containing calcium below or equal to normal blood serum ionized calcium levels have been used with hypercalcemic patients to reduce serum and total calcium levels but no reduction or "clearing up" of cardiovascular calcification could be achieved. Moreover, they are often difficult to tolerate since patients present soon symptoms of hypocalcaemia. In part of the cases, this treatment results in pathological decalcification of the skeleton (due to the non-selectiveness of this type of medically induced hypocalcaemia applied to soft-tissue calcification, i.e. outside the skeleton).

Prevention and treatment of atherosclerotic cardiovascular deposits in the general population and HD patients have been used so far dietary regimens and medication to reduce lipid absorption in the gastrointestinal tract and inhibit the formation or enhancing the decomposition and elimination of serum atherogenous lipid fractions via the faeces, as well as LDL aphaeresis. All these achieve some improvement of serum dyslipidaemia but are nearly ineffective in removing the atheromatous plaques in the cardiovascular structures.

Intravenous and oral administration of disodium ethylene diamine tetracetic acid (further referred to as DS-EDTA) are known in the medical practice as chelator of heavy metals and calcium. DS-EDTA intravenous infusion is widely used in toxicology to manage leading and other metal poisonings. It is also administered intravenously and orally to reduce atheromatous plaques. The therapeutic DS-EDTA concentrations used in the above cases have been proven to be harmless to the human body. Only doses highly above the therapeutic ones require additional administration of preparations containing magnesium, zinc, vitamin C and vitamins B that DS - EDTA also binds to but to a considerably lower degree than to heavy metals and calcium. The use of DS-EDTA in dialysis solutions is not known so far.

### SUMMARY OF THE INVENTION

The problem to be solved is to make concentrated solutions from which ready-for-use hemodialysis solutions can be prepared by dilution with water and then used in the prevention and treatment of cardiovascular and other pathologic soft-tissue calcification, as well as in the prevention and treatment of calciphylaxis and dealing with calcium body overload.

According to the invention, concentrated hemodialysis solutions containing DS-EDTA as an active substance dosed from 0.5 to 50.0 g/l or 1.487 - 148.700 mmol/l were made. More specifically are made.
(1) Concentrated acid solutions for bicarbonate hemodialysis are as follows:

| **Ions** | **Concentrated solutions for bicarbonate hemodialysis in mmol/I** |
|---|---|
| Sodium | from 4550.0 to 5075.0 |
| Potassium | from 00.00 to 105.0 |
| Calcium | from 00.00 to 70.0 |
| Magnesium | from 00.0 to 42.0 |
| Chlorine | from 3150.0 to 4200.0 |
| Acetate | from 87.5 to 350.0 |
| Glucose | from 00.00 to 420.0 |
| DS-EDTA | from 1.487 to 148.700 |

(2) A basic bicarbonate solution containing 1000.0 mmol/l of sodium ions, 1000.0 mmol/I of hydrogen carbonate ions and DS-EDTA from 1.214 to 121.388 mmol/l;
(3) Concentrated solutions for acetate hemodialysis with the following composition:

| **Ions** | **Concentrated solutions for acetate hemodialysis in mmol/l** |
|---|---|
| Sodium | from 4550.0 to 5075.0 |
| Potassium | from 00.00 to 105.0 |
| Calcium | from 00.00 to 70.0 |
| Magnesium | from 00.0 to 42.0 |
| Chlorine | from 3150.0 to 4200.0 |
| Acetate | from 1120.0 to 1575.0 |
| Glucose | from 00.00 to 420.0 |
| DS-EDTA | from 1.487 to 148.700 |

The concentrated solutions according to the invention are indented to be used for preparing ready-for-use solutions with various concentrations of ions and DS-EDTA depending on the patient's needs. The acid and hydrogen carbonate concentrated solutions according to the invention can also be used to make ready-for-use DS-EDTA containing solutions by adding them to a variety of known concentrated solutions. The solutions are aimed to patients with acute or chronic renal failure on hemodialysis to prevent and treat cardiovascular and other types of soft-tissue calcifications; for the prevention and treatment of calciphylaxis; for the elimination of calcium body overload; they make it possible without risk to increase the doses of calcitriol and calcium-containing phosphate binders (need for the prevention and treatment of renal osteodystrophy); they can be used in the prevention and treatment of atherosclerosis, including diabetic macroangiopathy and also in the management of heavy metal poisonings; they have an effect on the activation of the complement - a negative side effect of dialysis membranes manifested as they come into contact with the patient's blood at the beginning of each hemodialysis session - and allow to reduce the dose of direct anticoagulant (heparin) which is mandatory in hemodialysis.

DS-EDTA, due to its concentration gradient and molecular weight, passes through the dialysis membrane from the dialysis solutions into the patient's bloodstream. These solutions should replace conventional dialysis solutions for different durations of the dialysis session up to its full duration, depending on the amounts and types of existing soft-tissue calcific deposits, ionized calcium serum levels prior to dialysis session, and each patient's individual tolerance to DS-EDTA solutions. During the dialysis session, DS-EDTA enters the bloodstream and leaves it after about 60 minutes; it is distributed throughout the extravasal spaces where it stays in direct contact with pathologic soft-tissue calcifications, the calcifications in the atheromatous plaques, and some calcium-depending proteins on the cell surface (cathesins and lisins) promoting the role of monocytes and macrophages in pathologic calcification. DS-EDTA is slowly eliminated over the next 24 hours in patients with functioning kidney, while in patients with non functioning kidneys it is cleared up by hemodialysis with a clearance close to that of similar low-molecular substances since DS-EDTA has a molecular weight of 336.24 D., i.e. below 500 D which is the limit between low and medium molecules. (It is known that conventional hemodialysis eliminates mostly substances with a molecular weight below 500 D). It is proven that the DS-EDTA has the highest calcium-binding affinity under the specific pH and other conditions in the human body, i.e. it exhibits an affinity to chelate calcium in the medium found in the patient's body. Since it stays for a short time in the blood, DS-EDTA binds to a small amount of ionized serum calcium, and, as it stays much longer in the extravasal spaces, it binds much more to free extracellular and loose connected soft-tissue calcium (located extracellularly in the cardiovascular system, skin, muscles, periarticular tissues, eye structures, and all viscera, as well as in the atheromatous plaques). Mainly by extracting the soft-tissue and extracellular extravasal calcium, DS-EDTA destroys soft-tissue calcifications and prevents further calcification by binding to the basic substratum (calcium), thus blocking the activation of monocytes and macrophages and their secretion of osteogenic factors. Calcium extraction from the atheromatous plaques results in their destruction and in the release of atherogenous lipoproteins into the serum where they can be subjected to other antiatherogenous treatment methods more easily.

In intoxication with heavy metals, DS-EDTA binds to them though the well-known mechanism of chelation and they are eliminated by conventional hemodialysis. Adding DS-EDTA to the dialysis solution used to patient's detoxification with heavy metal poisoning and functioning kidneys through hemodialysis is better than intravenous application of DS-EDTA, because it spares the kidneys since they will not take part in the elimination of the DS-EDTA-metal complexes. Their excretory function is replaced by hemodialysis. In patients with non functioning kidneys, hemodialysis remains the only possible choice.

The application of DS-EDTA solutions for hemodialysis proposed -concentrated contained DS-EDTA - has not been used so far. In HD patients, it is more rational than the current practice of daily intravenous DS-EDTA infusion (in patients with atherosclerosis or metal intoxication), because it is time saving for the patient and the staff (intravenous infusion takes necessarily 1 to 3 hours) and, in addition to the effect of the usual dialysis procedure, it allows a more flexible, i.e. a longer and more adequate therapeutic approach to soft-tissue calcification, calcium body overload, atheromatous plaques, and heavy metal intoxications. Also, it helps to prevent complement activation at the beginning of the hemodialysis session and to reduce the dose of heparin used in hemodialysis. Hemodialysis solutions whose concentrated form contains DS-EDTA can be used in regular hemodialysis sessions which are performed 3 times per week but the weekly number of sessions can be increased if necessary to ensure an individual therapeutic dose and algorithm of administration. Dialysis should be performed successively with DS-EDTA-containing and conventional (DS-EDTA-free) concentrated solutions to allow the elimination of EDTA-calcium and EDTA-metal complexes. In this way it is possible to be release the body from the calcium accumulated abnormally through pathologic calcification or from the heavy metals. On the one hand, DS-EDTA acts on the plaques by binding to the calcium they accumulate and on the other hand, it improves the serum lipid transport to the liver where they are metabolized.

Moreover, no additional intravenous manipulation is needed - patients do not have to stay for the otherwise mandatory 1 to 3 hours beside dialysis to receive an intravenous infusion. At the same time, it becomes possible to administer a minimum i.e. non toxic and steady DS-EDTA concentration and to modify with a maximum flexibility the times of application of the new and conventional (DS-EDTA-free) solutions within the regular dialysis session or within the weekly dialysis schedule, depending on how well the patient tolerates the solution and also on the ionized calcium and DS-EDTA serum levels evaluated *ex tempore.* HD solutions which content DS-EDTA can be used in HD patients on a regular basis to prevent soft-tissue calcification with cardiovascular and any other localization. So far the other methods used to lower serum and total calcium levels in HD patients are based upon the principle of communicating vessels and thus induce hypocalcaemia which indirectly results in calcium extraction from the soft-tissue calcifications. The realisation of this secondary effect can be obtained in extreme hypocalcaemia which would induce dangerous symptoms such as paraesthesia, cramps, convulsions, breathing difficulty, and heart rhythm and conduction disorders. DS-EDTA directly chelates the loose connected calcium in the soft-tissue calcium deposits and atheromatous plaques, as well as the free extravasal calcium needed to activate pathologic extraskeletal osteogenesis, and, to a much lower and harmless degree, serum ionized calcium. [E. Cranton & J. Frackelton; Scientific Rationale for EDTA Chelation Therapy. Mechanism of Action. In: A Texbook on EDTA Chelation Therapy, Second Edition, 2001, edited by Elmer M. Cranton, M.D., Hampton Roads Company, Charlotesville, Virginia]. Owing to the nature of the above effects, DS-EDTA has rather a targeted effect on the decrease of soft-tissue calcifications than on the general decalcification effect which allows avoiding skeletal decalcification in the treated patient.

DS-EDTA is suitable for the prevention and treatment of calciphylaxis, a severe complication in HD patients involving calcification and inflammatory change in the walls of small limb arterial vessels that entails gangrenous necrosis and amputation. Here again, due to its rapid evacuation from the intravasal space and appropriate pH allowing selective calcium chelation, DS-EDTA binds mainly to calcium in the tissues and extravasal spaces and much less to serum ionized calcium.

The use of DS-EDTA concentrate solutions to make ready-for use solutions will allow the administration of calcium-containing oral phosphate binders in higher doses, which was necessary and safety but so far impossible, to prevent and treat hyperphosphataemia - a particularly relevant symptom of renal osteodystrophy and a cause underlying soft-tissue calcium deposits in CRF patients on hemodialysis.

The use of DS-EDTA concentrate solutions to make ready-for use solutions will facilitate a more successful calcitriol supplementing in all CRF patients and will allow a safe administration of calcitriol in higher therapeutic doses which is the major pathogenetic principle in preventing and treating high turnover renal osteodystrophy in CRF patients on HD related to secondary hyperparathyroidism (without risk of hypercalcaemia and soft-tissue calcium deposits).

Chelation of the free extravasal calcium and the calcium in the atheromatous plaques results in the reduction or full destruction of existing atheromatous plaques in the cardiovascular structures and prevents new plaques formation.

In heavy metal intoxication, DS-EDTA binds to the metal in the bloodstream through the well-known mechanism of chelation and is then eliminated by conventional hemodialysis using successively DS-EDTA-containing and DS-EDTA-free (conventional) solution. Adding DS-EDTA to the dialysis solution used to detoxificate patients with heavy metal poisoning and functioning kidneys through hemodialysis is better than intravenous application of DS-EDTA, because it spares the kidneys since they will not take part in the elimination of the DS-EDTA-metal complexes. Their excretory function is replaced by hemodialysis. However, hemodialysis remains the only possible choice for patients with non functioning kidneys.

The use of DS-EDTA concentrate solutions to make ready-for use solutions will prevent the activation of the complement - a negative side effect of dialysis membranes manifested as they come into contact with the patient's blood at the beginning of each hemodialysis session.

### PREFERRED EMBODIMENTS:

Preferred embodiments are given in the tables below, with the amounts of medicinal substance and salts being measured in (**g/l**)

### EXAMPLE Nº 1.

**Acid concentrated solution for bicarbonate hemodialysis with DS-EDTA:**

| | |
|---|---|
| Sodium chloride | 210.00 |
| Potassium chloride | 7.82 |
| CaCl₂.6H₂O | 13.45 |
| MgCl₂.6H₂O | 5.44 |
| Ice acetic acid | 4.21 |
| DS-EDTA | 7.00 |

Purified water -EurPh 2005 to 1000,00 ml

### EXAMPLE Nº2

**Bicarbonate concentrated hemodialysis solution with DS-EDTA:**

| | |
|---|---|
| NaHCO₃ | 84.00 |
| DS-EDTA | 5,714 |

Purified water - EurPh2005 to 1000,00 ml

### EXAMPLE Nº3

**Acid concentrated solution for acetate hemodialysis with DS-EDTA:**

| | |
|---|---|
| Sodium chloride | 200.68 |
| Potassium chloride | 7.82 |
| CaCl₂.6H₂O | 13.45 |
| MgCl₂·6H₂O | 5.44 |
| Sodium acetate. 3H₂O | 183.29 |
| DS-EDTA | 5.00 |

Purified water - EurPh 2005 to 1000,00 ml

### EXAMPLE Nº4

Ready-for-use solution prepared from a concentrated solution by a known method according to Example 1 mixed with hydrogen carbonate DS-EDTA-free solution for bicarbonate hemodialysis and containing the following ingredients (in mmol/l):

| | |
|---|---|
| Sodium | 140.0 |
| Potassium | 3.0 |
| Calcium | 1.5 |
| Magnesium | 0.56 |
| Chlorine | 112.5 |
| Acetate | 4.0 |
| Glucose | 6.0 |
| Hydrogen carbonate | 35.0 |
| DS-EDTA | 0.595 |

### EXAMPLE Nº5

Ready-for-use solution prepared by a known method from a concentrated solution according to Example 1 but not containing DS-EDTA mixed with DS-EDTA-containing hydrogen carbonate solution according Example 2 for bicarbonate hemodialysis containing the following ingredients (in mmol/l) :

| | |
|---|---|
| Sodium | 140.0 |
| Potassium | 3.0 |
| Calcium | 1.5 |
| Magnesium | 0.56 |
| Chlorine | 112.5 |
| Glucose | 6.0 |
| Acetate | 4.0 |
| Hydrogen carbonate | 35.0 |
| DS-EDTA | 0.595 |

### EXAMPLE Nº 6

Ready-for-use acetate hemodialysis solution prepared from a concentrated solution according to Example 3 and containing the following ingredients (in mmol/l):

| | |
|---|---|
| Sodium | 138.0 |
| Potassium | 3.0 |
| Calcium | 1.75 |
| Magnesium | 0.75 |
| Chorine | 107.6 |
| Acetate | 38.0 |
| Glucose | 6.0 |
| DS-EDTA | 0.425 |

## Claims

1. Concentrated hemodialysis solutions **characterized by** containing - in addition to sodium, calcium, potassium, magnesium, chorine, acetate, hydrogen carbonate ions and glucose - disodium ethylene diamine tetraacetic acid (DS-EDTA) as an active substance in a concentration ranging from 0.5 to 50.0 g/l or from 1.487 to 148.70 mmol/l.

2. Concentrated hemodialysis solutions, according to Claim 1, especially acid solutions used to prepare ready-for-use bicarbonate hemodialysis solutions **characterized by** containing ions in concentrations (in mmol/l) falling between the following limits:
| | |
|---|---|
| Sodium | from 4550.0 to 5075.0 |
| Potassium | from 0.00 to 105.0 |
| Calcium | from 0.00 to 70.0 |
| Magnesium | from 0.00 to 42.0 |
| Chlorine | from 3150.0 to 4200.0 |
| Acetate | from 87.5 to 350.0 |
| Glucose | from 0.00 to 420.0 |
| DS-EDTA | from 1.487 to 148.70 |

3. Concentrated hemodialysis solutions according to Claim 1 especially used to make ready-for-use bicarbonate hemodialysis solutions **characterized by** containing sodium hydrogen carbonate from 595.189 to 1050.0 mmol/l and DS-EDTA from 1.487 to 148.7mmol/l.

4. Concentrated hemodialysis solutions according to Claim 1 especially used to make ready-for-use acetate hemodialysis solutions **characterized by** containing ions within the following limits (in mmol/l):
| | |
|---|---|
| Sodium | from 4550.0 to 5075.0 |
| Potassium | from 00.00 to 105.0 |
| Calcium | from 00.00 to 70.0 |
| Magnesium | from 00.0 to 42.0 |
| Chlorine | from 3150.0 to 4200.0 |
| Acetate | from 1120.0 to 1575.0 |
| Glucose | from 00.00 to 420.0 |
| DS-EDTA | from 1,487 to 148,70 |

5. Using the concentrated solutions according to Claims 1 through 4 to make ready-for-use bicarbonate and acetate hemodialysis solution with the following ion composition:
For bicarbonate hemodialysis (in mmol/l
| | |
|---|---|
| Sodium | from 130.0 to 145.0 |
| Potassium | from 0.00 to 4.0 |
| Calcium | from 0.00 to 2.0 |
| Magnesium | from 0.00 to 1.2 |
| Chlorine | from 90.0 to 120.0 |
| Acetate | from 2.5 to 10.0 |
| Glucose | from 0.00 to 12.0 |
| Hydrogen carbonate | from 19.18 to 36.75 |
| DS-EDTA | from 0.042 to 4.248 |
For acetate haemodialysis (in mmol/l):
| | |
|---|---|
| Sodium | from 130.0 to 145.0 |
| Potassium | from 0.00 to 3.0 |
| Calcium | from 0.00 to 2.0 |
| Magnesium | from 0.00 to 1.2 |
| Chlorine | from 90.0 to 120.0 |
| Acetate | from 32.0 to 45.0 |
| Glucose | from 0.00 to 12.0 |
| DS-EDTA | from 0.042 to 4.248 |
for the prevention and treatment of cardiovascular diseases involving atheromatous change and cardiovascular and soft-tissue calcification in chronic renal failure patients on hemodialysis; for the prevention and treatment of calcium body overload in chronic renal failure patients on hemodialysis; for the prevention and treatment of calciphylaxis in chronic renal failure patients on hemodialysis; for the prevention and treatment of renal osteodystrophy (in the presence of hyperphosphataemia and hypercalcaemia) in chronic renal failure patients on hemodialysis; for the prevention and treatment of diabetic macroangiopathy in diabetic patients on hemodialysis; for the treatment of heavy metal intoxication in patients with general renal failure and chronic renal failure.
